# EUROPEAN PATENT APPLICATION

(11) **EP 3 093 024 A1**
(43) Date of publication of application: **16.11.2016**
(21) Application number: 15705897.5
(22) Date of filing: 08.01.2015
(51) Int. Cl.: A61K 38/18, A61K 47/48

(54) **CONJUGATE COMPRISING ERYTHROPOIETIN AND A BRANCHED POLYMER STRUCTURE**

(30) Priority: 08.01.2014 CU 20140003
(71) Applicant: Centro De Ingenieria Genetica Y Biotecnologia, La Habana 11600 (CU); Centro de Inmunologia Molecular, La Habana 11600 (CU)
(72) Inventor: PÁEZ MEIRELES, Rolando, 11600 La Habana (CU); AMARPO GONZÁLEZ, Daniel, Enrique, 11300 La Habana (CU); CASTRO ODIO, Fidel, Raúl, 10600 La Habana (CU); HERNÁNDEZ VALDES, Yenisel, 32200 Artemisa (CU); RUIZ ESTRADA, Gladys, Amalia, 10500 La Habana (CU)
(74) Representative: V.O.
(86) International application number: PCT/CU2015/000001
(87) International publication number: WO 2015/104008

(57) **Abstract**

The present invention discloses a conjugate comprising erythropoietin (EPO) and an asymmetric branched polymeric structure comprising two branches of monomethoxypolyethylene glycol (mPEG), where the molecular mass of one of these mPEG branches is between 10 kDa and 14 kDa, and the molecular mass of the other branch of mPEG is between 17 kDa and 23 kDa, as well as the pharmaceutical compositions containing it. The invention also provides a method for the preparation of pegylated EPO, wherein said protein is conjugated to an asymmetric branched polymeric structure with two branches of mPEG having the above described molecular masses.

## Description

### Technical field

The present invention relates to the field of biotechnology, life sciences and pharmaceutical industry, in particular to molecule modification to improve its pharmacokinetics, increase its half-life in blood and its biological activity.

### Background of the Invention

The use of biomolecules for therapeutics in humans has increased in recent years, mainly due to: (1) the discovery of new protein and peptide molecules, (2) better understanding of the mechanisms of action in *vivo,* (3) improvement in the expression systems of proteins and peptide synthesis and (4) improvement in the formulations or molecule modification technologies that enhance the pharmacodynamic and pharmacokinetic properties.

Technological developments in the field of modified drug delivery have allowed the introduction of a large number of systems that alter the release of injectable drugs, with the aim of improving the pharmacodynamic and pharmacokinetic properties of therapeutic agents. New drug delivery systems can be produced by a change in formulation (e.g., continuous product release or liposomes), or by an addition to the drug molecule, such as pegylation, which is only to covalently bind the drug to one or more molecules of polyethylene glycol (PEG).

New forms of release lead to increased half-life, decreased adverse effects, increase in drug efficacy, and improved quality of life for patients. Continuous release systems release the drugs in a controlled and predetermined manner, and are particularly suitable for drugs for which is important to avoid large fluctuations in plasma concentrations.

Systemic release of molecules using biodegradable microspheres has been widely studied due to the protection of proteins susceptible to degradation and to extended or modified release (Sinha, V.R. and Trehan, A. (2003). Biodegradable microspheres as protein delivery, J. Controlled Release, 90 (3): 261-280).

Pegylation provides a method for modification of therapeutic proteins to minimize many of the pharmacological limitations of biomolecules. For example, the half-life in blood increases for several reasons, including: the polymeric residue may prevent protease attack and recognition of the drug by the immune system, and the significantly higher hydrodynamic volume of the conjugate, relative to the native protein, significantly diminishes filtration by the kidney. Although in many cases the *in vitro* biological activity of a protein is affected by pegylation; the substantial increase in the blood lifetime makes its therapeutic action more effective (Harris J.M. and Chess R.B. (2003) Effect of pegylation on pharmaceuticals Nat Rev Drug Discov 2:214-221).

Another benefit of pegylation is given by the increased physical stability, since it sterically blocks the degradation pathways induced by hydrophobic interactions and generates non-specific sterical obstacles that diminish the intermolecular interactions involved in the thermal instability of proteins. Increased physical stability allows for more stable formulations (Harris J.M. and Chess R.B. (2003) Effect of pegylation on pharmaceuticals Nat Rev Drug Discov 2:214-221).

With the emergence of the second generation of activated PEG, groups that allowed more selective pegylation (for example: the aldehyde group that preferentially binds to the *N*-terminal end of proteins) and branched structures (Roberts M.J., Bentley M.D., Harris J.M. (2002) Chemistry for peptide and protein PEGylation Adv Drug Deliv Reviews. 54:459-476) became available. Branched PEGs developed include the two branched monofunctional (Patent no. US 5932462), four branched tetrafunctional, and eight branched octafunctional. For conjugation of therapeutic proteins, the monofunctional activated PEGs are more useful, because they avoid crosslinking between the protein and the PEG polymer. Branched PEGs also have an umbrella type effect, which allows better protection of the protein surface.

The two branched monofunctional PEG has allowed obtaining conjugated alpha-2a interferon that has shown better clinical results than the native protein (Rajender Reddy K., Modi M.W., Pedder S. (2002). Use of peginterferon alfa -2a (40 KD) (Pegasys) for the treatment of hepatitis C. Adv Drug Deliv Reviews. 54:571-586).

There are reports of a large number of proteins to which different modified release technologies have been applied to, including recombinant human erythropoietin (EPO), (rh EPO). EPO is a glycoprotein with 165 amino acids. Its estimated molecular weight using sodium dodecyl sulfate-polyacrylamide gel electrophoresis (abbreviated SDS-PAGE) is between 27 and 39 kDa (Mikaye, T. et al. (1977). Purification of Human erythropoietin J Biol Chem 252:5558-5564), depending on its degree of glycosylation.

In experiments, it has been demonstrated that by removing the hypoxic stimulus; the EPO messenger ribonucleic acid (mRNA) decreases rapidly in the blood and may be undetectable at 3 hours, indicating that its half-life is short (Lacombe, C. et al (1988) Peritubular cells are the site of erythropoietin synthesis in the murine hypoxic kidney J. Clin Ingest 81:620-623; Koury, S.T. et al. (1989) Quantitation of erythropoietin producing cells in kidneys of mice by in situ hybridation: Correlation with hematocrit, renal erythropoietin mRNA and serum erythropoietin concentration. Blood 74:645-651). It was estimated that the EPO half-life in blood plasma ranges between 4 and 13 hours; this time is very short, when compared with other molecules of therapeutic use. This causes that a patient requiring the use of the hormone regularly has to be frequently injected to maintain erythrocyte levels close to normal (Spivak, J.L. 1992 The mechanism of action of erythropoietin: Erythroid cell response. In J.W. Fisher (Ed.) Biochemical Pharmacology of Blood and Blood-Forming Organs Springer-Verlag Berlin Handbook of Experimental Pharmacology 101: 49-114; Jelkmann, W. (1986) Renal Erythropoietin: Properties and Production Rev Physiol Biochem Pharmacol 104:139-205).

With the objective of increasing the half-life of rh EPO, mutations have been carried out on the molecule to increase the sites of *N*-glycosylation (Burke, Paul (2003). Device for the sustained release of aggregation-stabilized, biologically active agent. Patent Application in the United States No. 20030133979), it has been encapsulated in microspheres (Morlock M, et al (1998). Erythropoietin loaded microspheres prepared from biodegradable LPLG-PEO-LPLG triblock copolymers: protein stabilization and in-vitro release properties. J Control Release, 56 (1-3): 105-115) and in liposomes (Moriya H, et al. (1997). Pharmacokinetic and pharmacological profiles of free and liposomal recombinant human erythropoietin after intravenous and subcutaneous administrations in rats. Pharm Res 14 (11): 1621- 1628), and a rh EPO dimer obtained has been reported to increase its biological activity (Bruno D., et al (2001). Dimeric erythropoietin fusion protein with enhanced erythropoietic activity in vitro and in vivo. Blood, Vol. 97, No. 12, 3776-3782).

One of the modifications having most results in clinics is the chemical conjugation of rh EPO with polymers (Jolling K. et al. (2005). Mixed-Effects Modelling of the Interspecies Pharmacokinetic Scaling of Pegylated Human Erythropoietin. Eur J Pharm Sci; 24: 465-475). The difficulty found in the development of this rh pegylated EPO has been the low yield of the conjugation process, due to the formation of monopegylated and bipegylated species, the latter constituting a contaminant of the process.

For all the above mentioned, it is still of interest to obtain new conjugates of EPO and polymers, which offer therapeutic advantages over the unmodified molecule.

### Description of the Invention

The present invention solves the problem stated above, by providing a conjugate comprising EPO and an asymmetric branched polymeric structure comprising two branches of monomethoxy polyethylene glycol (mPEG), where the molecular mass of one of these mPEG branches is between 10 kDa and 14 kDa, and the molecular mass of the other branch of mPEG is between 17 kDa and 23 kDa.

In this way, the invention provides a conjugate comprising a monofunctional branched PEG structure with a molecular size similar to that used by Papadimitriou (US Patent no. 7202208), but which employs a structure of two PEG strands of different molecular masses. This alternative, unexpectedly, confers new advantages to the drug. In the conjugate of the invention, the total molecular mass of the two branched PEG polymer structure is between 27 and 37 kDa. In one embodiment of the invention, the molecular mass of mPEG1 is 12 kDa and the molecular mass of mPEG2 is 20 kDa.

The use of asymmetric branched PEG chains, instead of linear ones, which have the indicated molecular mass allowed to reduce the formation of bipegylated contaminants, and unexpectedly significantly increased the half-life of the molecule, and its physicochemical stability.

Another unexpected result was that the biological activity of the pegylated molecule with the asymmetric branched monofunctional PEG remained intact. The loss of biological activity of biomolecules caused by pegylation has been widely reported in the literature (Harris JM and Chess RB (2003) Effect of pegylation on pharmaceuticals Nat Rev Drug Discov 2:214-221). Monofunctional PEG with two asymmetric branches is obtained by the binding of two linear PEG chains with different molecular size to a core. A similar process has been used by other authors with good results (US Patent no. 5932462). To bind the two linear PEG chains to a core required them to have an active group. This group can be selected from various groups known in the art. For example, this active group can be succinimidyl succinate, succinimidyl carbonate, p-nitrophenylcarbonate, succinimidyl propanoate, succinimidyl butanoate, among others. A linear PEG, preferred in this invention, is activated with succinimidyl carbonate. This is due to two main reasons: a) the good yields of the reaction between it and the amino groups and b) the ease of the process for obtaining this functionalized PEG. The process of obtaining this functionalized PEG is known to those working in this technical field (Miron T, Wilchek M. (1993). A Simplified Method for the Preparation of Succinimidyl Carbonate Polyethylene Glycol for Coupling to Proteins. Bioconjugate Chem. 4:568-569). Once the linear PEG is activated, it is subsequently allowed to react with the selected core molecule.

In a preferred embodiment of this invention the core is L-lysine, since it is a biocompatible molecule, with two free amino groups and a carboxylic group that can be used to be activated later. Therefore, in one embodiment of the invention, EPO is conjugated to an asymmetric branched polymer structure which is represented as:

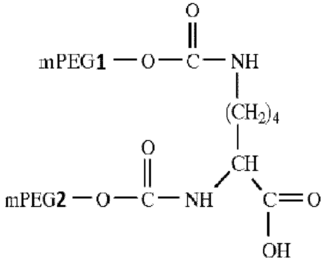

In a particular embodiment, in said polymeric structure, forming part of the conjugate, the molecular mass of mPEG1 is 12 kDa and the molecular mass of mPEG2 is 20 kDa, or the molecular mass of mPEG1 is 20 kDa and the molecular mass of mPEG2 is 12 kDa.

The derivative of two asymmetric branches can be purified using chromatographic methods, and subsequently activated using different reactive groups for conjugation to biomolecules. Any of the functional groups used for the activation of other PEG structures can be used for the asymmetric branched PEG described in this invention. Examples of these functional groups are: *N*-hydroxysuccinimide esters, succinimidyl carbonate, different types of aldehydes, maleimides, among others. Another type of functional groups that allow the binding of this structure to proteins are the chelating groups, such as nitrilotriacetate, which by means of a transition metal can be conjugated to the histidines present in the peptide backbone. The choice of the reactive group to be used depends on the residue of the protein to which PEG is bound.

In the examples of the patent application filed by Huang (EP 1479711 A1), PEG branched structures are shown with different molecular masses in each of the branches; but the molecular size of the branches is smaller than that of the present invention. In patent application No. EP 1479711 A1, the inventors suggest that the biological activity of the conjugate decreases with respect to the biological activity of the unmodified drug. However, in the present invention, unexpectedly, the biological activity of EPO conjugated with monofunctional PEG with two asymmetric branches, of total molecular mass between 27 and 37 kDa, was similar to that of the unmodified EPO. On the other hand, in the present invention it was shown that the half-life of a PEG structure with two branches, which have lower molecular masses, according to the patent application filed by Huang, is less than the half-life time of the EPO conjugate with PEG of two branches of 12 kDa and 20 kDa, respectively, of this invention.

This result obtained in the present invention was unexpected. With the conjugate comprising an asymmetric polymeric structure (PEG_{2,32K}) a spatial distribution of the molecule that does not limit the biological activity, improves the conjugation reaction decreasing the formation of polypegylated products, and significantly improves the pharmacokinetic parameters is achieved. This allows the reduction of the treatment dose for people who need it.

The basic raw materials for obtaining the asymmetric two branched PEG structure in the present invention are the 12 kDa mPEG (12K PEG) and 20 kDa mPEG (20K PEG). The molecular weight of these mPEG has a range established by the manufacturer, since PEG is a polydisperse polymer. For example, as specified by one of the manufacturers, polydispersity should be lower than 1.1%. In that case, the molecular weight range reported by the manufacturer for the 12K PEG would be 12.0 ± 1.2 kDa; and PEG 20K would be 20.0 ± 2.0 kDa.

To determine whether this difference in molecular weight between each batch of PEG initial material influences the pharmacokinetics results, an experiment with batches of PEG having molecular weights corresponding to the extreme values of the specifications (Example 11) was performed. A 12K PEG with 11 kDa molecular weight and 20K PEG with 18 kDa molecular weight were used to form an EPO conjugate having a two branched PEG structure of 29 kDa total molecular weight (PEG_{2,29K}-EPO). A 12K PEG with 13 kDa molecular weight and 20K PEG with 22 kDa molecular weight were also used to form an EPO conjugate having a two branched PEG structure of 35 kDa total molecular weight (PEG_{2,35K}-EPO). The results showed that the pharmacokinetic parameters are similar when different initial batches of 12K PEG and 20K PEG are used. Therefore, conjugates formed by the PEG_{2,29K}-EPO and PEG_{2,35k}-EPO structures are considered essentially the same as those formed by the structure with theoretical weight indicated by the manufacturer, which is the conjugate of EPO and two branched PEG, one of 12 kDa and another of 20 kDa (PEG_{2,32K}-EPO). Conjugation of the protein with activated PEG takes place within an appropriate buffer. The characteristics of the buffer depend, among other factors, on the functional group of the polymer and the objective of the conjugation. For example, if conjugation by the free amino groups with a functionalized PEG as *N*-hydroxysuccinimide ester is wanted, the conjugation sites can be predicted to some extent, depending on the pH of the conjugation reaction. A pH between 8.0 and 9.0 promotes conjugation by the ε-amino group of lysines.

After obtaining the conjugate, it is characterized using various techniques. The chemical, physical and biological properties of the conjugates are analyzed to achieve the fullest possible characterization of the purified conjugate. For example, the concentration of the conjugate can usually be determined by ultraviolet spectroscopy (280 nm absorbance), since the PEG residue practically does not affect the molar extinction coefficient of the protein. Purity of the purified product is preferably determined by SDS-PAGE, since chromatographic methods, like gel filtration chromatography, poorly discriminate the signals corresponding to the conjugate of interest and the contaminants. Other physico-chemical properties can be studied by the usual methods known to those skilled in the art.

In the context of this invention the term erythropoietin (EPO) refers to any variant of the EPO molecule that maintains its biological activity; for example, truncated molecules. EPO may be obtained by recombinant deoxyribonucleic acid (DNA) technology, using the expression and purification systems known by those skilled in this technical field. Therefore, in an embodiment of the present invention, the conjugate comprises rh EPO. The conjugate of the present invention also comprises any variant of EPO obtained by the methods described above, after being modified by any method of the prior art, such as amino acid substitution.

A pharmaceutical composition comprising any of the PEG-EPO conjugates disclosed herein and a pharmaceutically acceptable excipient is also an object of the present invention.

Another aspect of the present invention is a method for obtaining pegylated EPO wherein said protein is conjugated to an asymmetric branched polymeric structure comprising two mPEG branches, where the molecular mass of one of the mPEG branches is between 10 kDa and 14 kDa, and the molecular mass of the other branch of mPEG is between 17 kDa and 23 kDa. Through the method of the invention, an increase in the EPO half-life in the blood of a mammal, to which the protein is administered, compared to the half-life of unconjugated EPO is produced. Therefore, the invention provides a method for improving the pharmacokinetics of the protein, for the purpose of reducing the doses administered to a subject in need thereof. In the context of the invention, improvement of the EPO pharmacokinetic parameters should be understood as an increase in half-life and/or the mean residence time of said protein. Thus, the invention discloses a method of chemical modification of the EPO molecule, in order to increase the half-life of said molecule, wherein said protein is conjugated to an asymmetric branched polymeric structure comprising two mPEG branches, where the molecular mass of one of these mPEG branches is between 10 kDa and 14 kDa, and the molecular mass of the other mPEG branch is between 17 kDa and 23 kDa.

In one embodiment of the invention, the molecular mass of one of the mPEG branches of the asymmetric branched polymer structure is 12 kDa and the molecular mass of the other mPEG branch is 20 kDa. In one embodiment of the invention, the asymmetric branched polymer structure conjugated to the EPO, in the method of the invention, is represented as:

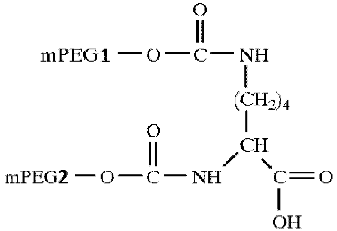

In a preferred embodiment of the method of the invention, in the asymmetric branched polymer structure that is conjugated to the EPO, the mPEG1 molecular mass is 12 kDa and mPEG2 molecular mass is 20 kDa, or the mPEG1 molecular mass is 20 kDa and the mPEG2 molecular mass is 12 kDa. In a more preferred embodiment, in the method of the invention, the EPO is rh EPO.

### Brief description of the Drawings

**Figure 1****.** SDS-PAGE double staining (Coomassie Brilliant Blue and iodine) results for the product of the conjugation reaction of the EPO with PEG_{2,32K}.
Sample 1 corresponds to the EPO positive control and sample 2 to the product of the conjugation reaction of the EPO with PEG_{2,32K}.
**Figure 2****.** SDS-PAGE double staining (Coomassie Brilliant Blue and iodine) results for the product of the conjugation reaction of the EPO with PEG_{2,40K}.
Sample 1 corresponds to the EPO positive control and sample 2 to the product of the conjugation reaction of the EPO with PEG_{2,40K}.
**Figure 3****.** Chromatograms obtained by gel filtration chromatography to evaluate different PEG-EPO conjugates.
**Figure 4****.** *In vivo* biological activity of the native EPO and the monopegylated EPO determined by the method of normocytic mice.
**Figure 5****.** Results obtained by the trypsin degradation assay of conjugates PEG_{2,32K} -EPO, PEG_{2,40K}-EPO and unmodified EPO. The degradation kinetics followed by SDS-PAGE is represented.
**Figure 6****.** Biological activity of different PEG-EPO conjugates and unmodified EPO, determined by the method of normocytic mice.

### Examples

### Example 1. Preparation of activated PEG as N-hydroxysuccinimide ester

### Activation reaction of mPEG_{20K}-OH

Fifty grams of mPEG of 20 kDa molecular weight without activation (mPEG_{20K}-OH) were dried azeotropically in 450 mL of toluene for 3 hours. After this time, 200 mL of solvent were removed and the solution allowed cooling to room temperature. Subsequently, 60 mL of dry dichloromethane (DCM) were added as cosolvent. Disuccinidimyl carbonate (4.9 g) was weighed and dissolved in 40 mL of dimethylformamide. The above solution was added to the flask containing the mPEG_{20K}-OH. Dimethylaminopyridine (2.5 g) was weighed and dissolved in 20 mL of DCM. The above solution was added to the flask containing the mPEG_{20K}-OH and stirring begun. It was allowed to react overnight at room temperature.

The reaction mixture was filtered to remove the solid products, using a glass fiber membrane. The filtrate was precipitated with 1.5 L of dry diethyl ether and filtered under vacuum. The precipitate was collected by vacuum, dried under high vacuum for 4 h and stored at -20 °C. Of the initial mass of mPEG_{20K}-OH, 96% was recovered as PEG succinimidyl carbonate (PEG_{20K}-SC) with a degree of activation of 96.0 ± 0.8%.

### Activation reaction of mPEG_{12K}-OH

The same procedure used for the activation reaction of mPEG_{20K}-OH was followed, but in this case, non activated mPEG of molecular weight 12 kDa (mPEG_{12K}- OH) was used as starting raw material.

### Reaction of L-lysine with PEG_{20K}-SC and PEG_{12K}-SC

L-lysine (9.1 g) was weighed and dissolved in 30 mL of 0.1 M boric acid; pH 8.0. Twenty grams of PEG_{20K}-SC were added to 15 mL of L-lysine solution in boric acid. The solution was kept under stirring for 12 hours.

Subsequently, the reaction mixture was diluted with 300 mL of distilled water and adjusted to pH 3.5 with a mixture of hydrochloric acid solution. The mixture was transferred to a separatory funnel, where 100 mL of DCM were added. It was stirred manually and the lower phase (organic phase) was removed. A total of 5 extractions were performed. Twenty grams of sodium sulfate were added to the bottle containing the organic phase and stirred manually until the phase was totally clear. It was vacuum filtered using a glass fiber membrane. The filtrate was concentrated to the maximum possible on the rotary evaporator. Then, 500 mL of diethyl ether were added and stirred manually, to precipitate the product. It was vacuum filtered and the product (20K monopegylated L-lysine) was dried for 1 h under vacuum in the rotary evaporator.

In the second reaction step, 9 g of PEG_{12K}-SC were added to 15 g of 20K monopegylated L-lysine. The reactants were dissolved in 215 mL of DCM and 0.14 mL of triethylamine added. The reaction mixture was vacuum filtered using a glass fiber membrane. The filtrate was concentrated on the rotary evaporator to maximum. Then, 300 mL of diethyl ether were added and stirred manually for 2 minutes to precipitate the product. It was filtered under vacuum and the product obtained (PEG with two asymmetric branches, one of 12 kDa and another of 20 kDa (PEG_{2,32K}-COOH)) was dried for 1 h under vacuum in rotary evaporator. The total yield of the process was higher than 70%.

### Purification of PEG_{2,32k}-COOH

Purification of PEG_{2,32K}-COOH was performed in a DEAE-Sepharose column of 80 cm height, using a volume flow of 40 mL/min. The gel was previously sanitized with 4.5 L of solution of 0.2 M sodium hydroxide. The column was equilibrated with a solution of 50 mM boric acid, pH 9.0. Subsequently, the column was washed with 5 L of distilled water. The PEG_{2,32K}-COOH sample dissolved in water was applied, to a conductivity of 40 mS/cm more than the conductivity of water. After applying the sample, the column was washed with 3 L of purified water. Sample elution was performed with 5 L of a solution of 10 mM sodium chloride. Fractions of 500 mL were collected. Subsequently, the column was regenerated with 3 L of a solution of 1M sodium chloride. The total yield recovered from the process was higher than 90%, with purity higher than 95%.

### Preparation of branched activated PEG as ester of N-hydroxysuccinimide (PEG_{2,32K}-NHS)

One gram of PEG_{2,32K}-COOH was dissolved in DCM; 0.01 g of *N-*hydroxysuccinimide (NHS) and 0.02 g of dicyclohexylcarbodiimide were added. The reaction mixture was stirred for 12 h. Subsequently, it was diluted with 5 mL of DCM and filtered through a glass fiber membrane. The filtrate was concentrated on the rotary evaporator and added to 400 mL of diethyl ether to precipitate the product. The mixture was vacuum filtered and the solid product (PEG_{2,32K}-NHS) collected and dried for 1 h under vacuum in the rotary evaporator. Activation degrees higher than 95% and recovery of the initial polymer mass above 90% were achieved.

### Example 2. Preparation of EPO conjugated with PEG_{2,32K}-NHS

### Conjugation reaction

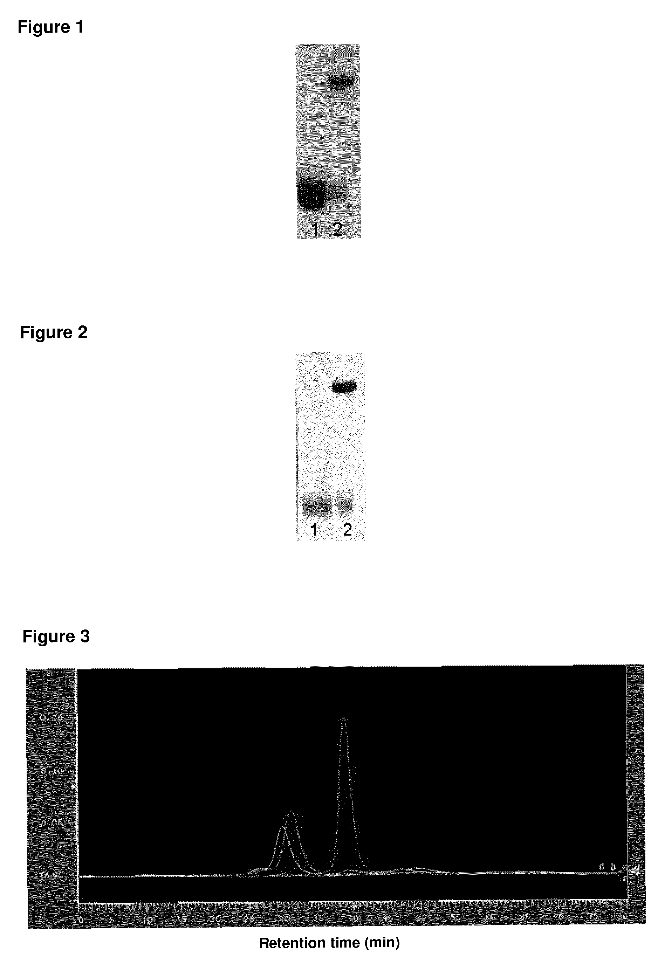

Six grams of PEG_{2,32K}-NHS were added to a solution containing 1 g of rh EPO at an initial concentration of 5 mg/mL in 50 mM borate solution, pH 8.0. The reaction was maintained for 2 hours at 4 °C with slow stirring. The reaction was stopped by diluting to a final protein concentration of 0.9 mg/mL with 50 mM boric acid, pH 8.0. The reaction yield was determined by densitometry of the SDS-PAGE gel, as shown in Figure 1. Total recovery was greater than 40%, with less than 3% of polypegylated products.

### Purification of the monopegylated EPO

To separate the products of the conjugation reaction of rh EPO with PEG, that is monopegylated EPO from free EPO and bipegylated EPO, a purification plan by anion exchange chromatography was designed. The separation process was conducted using a column packed with Q-Sepharose of 12 cm height. The gel was previously sanitized with 22 mL of 0.2 M sodium hydroxide. Then, 33 mL of purified water were passed and later, 33 mL of the equilibrium solution of 50 mM boric acid, pH 8.0. The sample from the conjugation reaction, diluted in equilibrium solution to a concentration of 0.9 mg/mL, was applied. Elution of the sample containing the EPO conjugate with PEG_{2,32K}-NHS (PEG_{2,32K}-EPO) is sequentially performed, using the same equilibration buffer with increasing concentrations of sodium chloride from 0.175 M to 0.5 M. The volumetric flow was 1.2 mL/min and the load, 0.58 mg of protein/mL of gel. The process recovery was 40% and the degree of purity, 97%.

### Example 3. Preparation of branched activated PEG as N-hydroxysuccinimide ester (PEG_{2,40K}-NHS)

To obtain PEG_{2,40K}-NHS, the procedure described in Example 1 was followed, but in the second step of the reaction with lysine, PEG_{20K}-SC was used. A total yield of more than 50%, with higher than 90% activation was achieved.

### Example 4. Preparation of EPO conjugated with PEG_{2,40K}-NHS

### Conjugation reaction

Six grams of PEG_{2,40K}-NHS were added to a solution containing 1 g of rh EPO at an initial concentration of 5 mg/mL in 50 mM borate buffer, pH 8.0. The reaction was maintained for 2 hours at 4 °C with slow stirring. The reaction was stopped by diluting to a final protein concentration of 0.9 mg/mL with 50 mM boric acid, pH 8.0. The reaction yield was determined by densitometry of the SDS-PAGE gel, as shown in Figure 2. The total recovery was higher than 40%, with less than 1% of polypegylated products.

### Purification of the monopegylated EPO

To separate the products of the conjugation reaction of the EPO with PEG, that is the monopegylated EPO from free EPO and bipegylated EPO, a purification plan by anion exchange chromatography was designed. The separation process was conducted using a column packed with Q-Sepharose, 12 cm in height. The gel was previously sanitized with 22 mL of 0.2 M sodium hydroxide; then 33 mL of distilled water were passed, and later 33 mL of the equilibrium solution of 50 mM boric acid, pH 8.0. The sample from the conjugation reaction, diluted in equilibrium solution to a concentration of 0.9 mg/mL, was applied. Elution of the sample containing EPO conjugated with PEG_{2,40K}-NHS (PEG_{2,40K}-EPO) was sequentially done using the same equilibration buffer with increasing concentrations of sodium chloride from 0.175 M to 0.5 M. The volumetric flow was 1.2 mL/min, and the load was 0.58 mg of protein/mL of gel. The process recovery was 40% and a 97% degree of purity was obtained.

### Example 5. Physicochemical characterization of the pegylated EPO

### Determination of the conjugate concentration

Conjugate concentration was determined by measuring absorbance at 280 nm in a spectrophotometer. The calculation of the protein concentration was performed using a molar extinction coefficient of 0.743 for EPO.

### Characterization by gel filtration chromatography

Chromatographic separations were performed using a high performance liquid chromatography (HPLC) pump and a UV detector with 226 nm filter. Application mass was 100 µg of protein. The chromatographies were performed with Superdex-200 matrix. Two variants of pegylated EPO (PEG_{2,32K}-EPO and PEG_{2,40K}-EPO) and unmodified EPO were analyzed. Figure 3 shows the correspondence between the estimated molecular mass for each variant and the resulting retention times. With the shorter retention time, PEG_{2,40K}-EPO conjugate was eluted, since it has the larger molecular size, then PEG_{2,32K} -EPO conjugate was eluted and finally, the unmodified EPO.

### Example 6. Biological characterization of the PEG_{2,32K}-EPO conjugate

The determination of the pegylated EPO potency was done by the method of normocytic mice. Mice of the B6D2F1 line (16-18 g) female, virgin, were inoculated subcutaneously with 0.2 mL of different dilutions (300, 450 and 600 IU/mL, equivalent to 3, 4.5 and 6 µg/mouse) of the samples of PEG_{2,40K}-EPO, PEG_{2,32K}-EPO, reference material (as positive control) and negative control (diluent only) at the rate of three animals per group. Two animals were additionally used, which were inoculated with a solution of 50 mM borate buffer, pH 8.0, to evaluate its toxicity. After 72 hours of the inoculation, a blood sample was collected by retro-orbital puncture from each animal, and deposited in vials containing 4 µL of sodium heparin (5000 IU). From peripheral blood, 40 µL were collected and mixed with 120 µL of a solution formed by 0.3 mM methylene blue solution, 1.29 mM sodium citrate, 5 mL of physiological saline, and 10 mL of distilled water. They were incubated for one hour in water bath at 37 °C. Afterwards, selective hemolysis was performed for 6 minutes by treatment with a lysing solution consisting of 0.08 mM of tetrasodium EDTA; 0.15 mM ammonium chloride; and 9.98 mM sodium bicarbonate. Erythrocyte lysis was stopped by overdilution with saline solution, and reticulocytes were visually counted in a Neubauer chamber. Data processing and potency calculations were performed using a random design of parallel lines and an unknown pattern, for detecting the validity of linearity and parallelism at a significance level of α = 0.05. The values obtained must comply with the provisions of the European Pharmacopoeia, which states that the value of the ratio of the calculated potency on the assumed one, expressed in percentage, should not be less than 80% nor more than 125%, and the fiducial limits of the calculated potency are to be in a range between 64% and 156%.

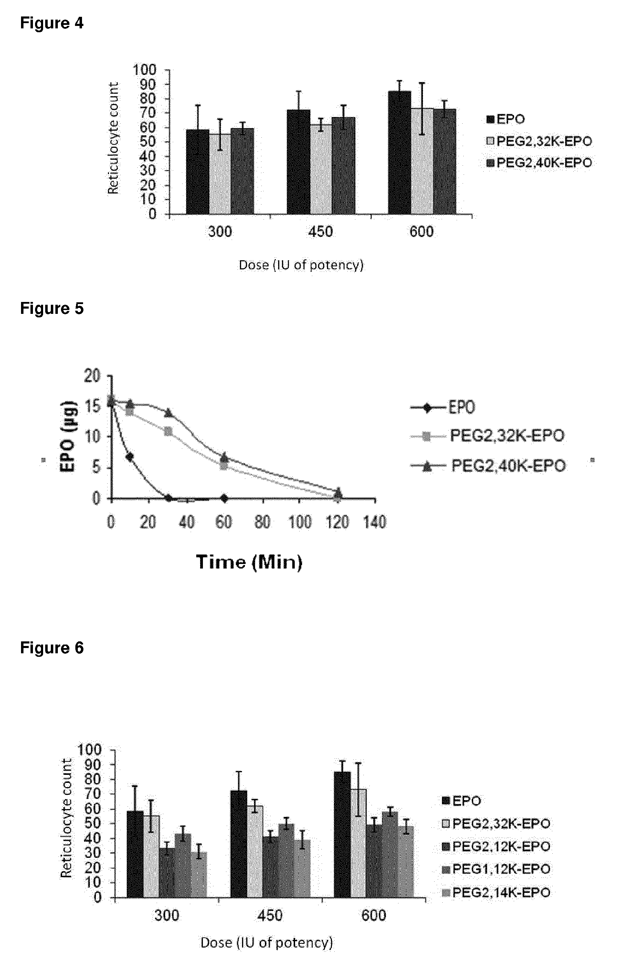

In Figure 4, the graphic can be observed of the data obtained directly from the reticulocyte count experiment where increasing EPO amounts were applied, measured in IU of potency. A statistical Tukey-Kramer test for comparison of means was applied, with the aim of analyzing the in *vivo* activity values. Statistical analysis of the data showed no significant differences between the values for *P<0.05.* The differences in the biological activities between pegylated EPO and unmodified EPO used as positive control were within the variation range of the assay (± 50%).

It is important to emphasize that the doses used to perform the assay had 300, 450 and 600 IU of potency. These are the doses established for detecting the biological activity of native EPO, whereas the assay conducted using the product of the Amgen Company, Mircera ™, which is an EPO conjugated with a linear polymeric structure of molecular mass 30 kDa (PEG_{1,30K}-EPO), requires a minimum dose of 6000 IU of potency. However, in the test conducted in this invention, with a dose 20 times lower, *in vivo* biological activity of the pegylated EPO was detected.

### Example 7. Resistance to protease degradation

To verify resistance to degradation by proteases of the different PEG-EPO molecules, an *in vitro* experiment was performed, in which the pegylated EPO and the native protein (as control) were digested with trypsin. Both pegylated EPO variants showed greater resistance to protease degradation than native EPO. In Figure 5, the results of the degradation kinetics of EPO used as control are shown, and the two pegylated variants PEG_{2,32K} -EPO and PEG_{2,40K} -EPO. It can be seen that the two pegylated EPO variants exhibit greater resistance to degradation by proteases than native EPO.

### Example 8. Pharmacokinetics of the PEG_{2,32K}-EPO conjugate

The pharmacokinetic study was done by comparing the unmodified EPO, the PEG_{2,32K}-EPO conjugate, the PEG_{2,40K}-EPO conjugate and the PEG_{1,30K}-EPO conjugate. Rabbits of the New Zealand strain with 2 kg mass were used. The results of half-life time (t ½), area under the curve (abbreviated AUC) and mean residence time (abbreviated MRT) are shown in Table 1

**Table 1. Comparative pharmacokinetics of EPO, PEG_{2,32K}-EPO conjugate, PEG_{2,40K}-EPO conjugate, and PEG_{1,30K}-EPO conjugate**

| **Pharmacokinetic parameter** | **PEG_{2,32K}**-**EPO** | **PEG_{2,40K}**-**EPO** | **PEG_{1,30K}**-**EPO** | **Unmodified EPO** |
|---|---|---|---|---|
| **t ½ (h)** | 165.93 +/-74.41 | 131.24 +/-6.70 | 68.33 +/-5.42 | 4.86 +/- 1.27 |
| **AUC (IU/mL·h)** | 83.59 +/-44.63 | 23.32 +/-14.92 | 242.44 +/-95.53 | 15.89 +/-2.31 |
| **MRT (h)** | 238.94 +/-12.05 | 199.62 +/-4.22 | 95.87 +/-15.89 | 6.62 +/- 1.95 |

It can be observed that all pegylated EPO had longer half-life time than the unmodified EPO. The half-life time of the PEG_{2,32K}-EPO conjugate was much higher (2.4 times) than that obtained for PEG_{1,30K}-EPO. This result is unexpected, considering that both conjugates exhibit very similar molecular sizes. This asymmetric branched molecule also exhibited a longer half-life time than the PEG_{2,40K}-EPO conjugate, which is also branched and has higher molecular weight, which was not expected either.

### Example 9. Comparison of the pharmacokinetic results of different EPO conjugates

This pharmacokinetic study was conducted comparing the PEG_{2,32K}-EPO conjugate, which has a branch of molecular mass 12 kDa and another of 20 kDa; EPO conjugated with an asymmetric PEG structure with a branch of molecular mass 5 kDa and another of 7 kDa (PEG_{2,12K}-EPO); EPO conjugate with a symmetrical structure of two branches of 7 kDa each (PEG_{2,14K}-EPO); and EPO conjugated to a linear PEG of 12kDa molecular mass (PEG_{1,12K}-EPO). For this study, the PEG_{2,12K}-EPO conjugate was obtained in a similar way to the PEG_{2,32K}-EPO conjugate, but using mPEG of lower molecular mass. The PEG_{2,14K}-EPO conjugate was similarly obtained to the PEG_{2,40K}-EPO conjugate, but using mPEG of lower molecular mass. The PEG_{1,12K}-EPO conjugate was obtained by activating the mPEG of 12 kDa molecular mass as described at the beginning of Example 1, and conjugation with the hr EPO was performed afterwards, as described in Example 2. Rabbits of the New Zealand strain, with mass of 2 kg, were used for the comparison. The results are shown in Table 2. The conjugate of the present invention (PEG_{2,32K}-EPO) showed longer half-life time than the rest of the conjugates analyzed.

**Table 2. Pharmacokinetic parameters of different EPO conjugates**

| **Parameter** | **PEG_{2,32K}-EPO (asymmetric, 12 and 20 kDa)** | **PEG_{2,12K}-EPO (asymmetric , 5 and 7 kDa)** | **PEG_{1,12K}-EPO** | **PEG_{2,14K}-EPO** |
|---|---|---|---|---|
| **t ½ (h)** | 165.93 +/- 74.41 | 89.72 +/- 7.87 | 38.33 +/- 10.97 | 81.63 +/- 10.31 |
| **AUC (IU/mL·h)** | 83.59 +/- 44.63 | 31.89 +/- 10.57 | 110.32 +/- 87.04 | 28.12 +/- 7.71 |
| **MRT (h)** | 238.94 +/- 12.05 | 87.14 +/- 17.54 | 46.41 +/-17.82 | 80.01 +/- 14.93 |

### Example 10. Comparison of the biological activity of different EPO conjugates

The biological activity of the conjugates was measured in a similar way to that described in Example 6. The results obtained for the conjugates: (1) PEG_{2,32K}-EPO; (2) PEG_{2,12K}-EPO; (3) PEG_{2,14K}-EPO; and (4) PEG_{1,12K}-EPO were compared. Unmodified rh EPO was used as control. In Figure 6 it can be seen that the conjugate of the present invention (PEG_{2,32K}-EPO) maintains the biological activity of the unmodified protein, unlike the rest of the conjugates analyzed, in which the biological activity decreases upon pegylation.

### Example 11. Pharmacokinetic study using the extreme limits of molecular mass of mPEG that form the two asymmetric branches of the PEG_{2,32K}-EPO conjugate

Since PEG is polydisperse, and the starting material used to form the structure of the asymmetric PEG, PEG_{2,32K} may have variation from the theoretical value of 12 kDa and 20 kDa of molecular weight, a pharmacokinetic study of EPO conjugates with PEG asymmetric structures with molecular masses of 29 kDa (PEG_{2,29K}-EPO) and 35 kDa (PEG_{2,35K}-EPO) was carried out. These are the limits of total molecular mass of the PEG forming part of the EPO conjugate of the present invention. The PEG_{2,29K}-EPO and PEG_{2,35K}-EPO conjugates were obtained in a similar manner to the PEG_{2,32K}-EPO conjugate. Rabbits of the New Zealand strain with mass of 2 kg were used. The results are shown in Table 3. As it can be observed, there are no differences in the pharmacokinetic parameters of the studied conjugated variants.

**Table 3. Results of the pharmacokinetic parameters of conjugates with PEG of different molecular weights**

| **Parameter** | **PEG_{2,32K}-EPO** | **PEG_{2,29K}-EPO** | **PEG_{2,35K}-EPO** |
|---|---|---|---|
| **t ½ (h)** | 165.93 +/- 74.41 | 159.24 +/- 19.84 | 174.01 +/- 25.66 |
| **AUC (UI/mL·h)** | 83.59 +/- 44.63 | 79.76 +/- 21.67 | 85.55 +/- 34.75 |
| **MRT (h)** | 238.94 +/- 12.05 | 243.42 +/- 18.54 | 235.34 +/-12.43 |

## Claims

1. A conjugate comprising erythropoietin (EPO) and an asymmetric branched polymeric structure comprising two branches of monomethoxy polyethylene glycol (mPEG), where the molecular mass of one of the branches of mPEG is between 10 kDa and 14 kDa, and the molecular mass of the other branch of mPEG is between 17 kDa and 23 kDa.

2. The conjugate according to claim 1 wherein the molecular mass of one of the branches of mPEG is 12 kDa and the molecular mass of the other mPEG branch is 20 kDa.

3. The conjugate according to claim 1 wherein the branched polymeric asymmetric structure is represented as:

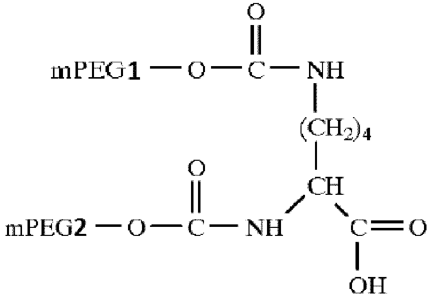

4. The conjugate according to claim 3 wherein the mPEG1molecular mass is 12 kDa and the mPEG2 molecular mass is 20 kDa, or the molecular mass of mPEG1 is 20 kDa and the mPEG2 molecular mass is 12 kDa.

5. The conjugate according to any one of claims 1-4 wherein the EPO is recombinant human EPO (rh EPO).

6. A pharmaceutical composition comprising the conjugate of any one of claims 1-5 and a pharmaceutically acceptable excipient.

7. A method for obtaining pegylated erythropoietin (EPO) wherein said protein is conjugated to an asymmetric branched polymeric structure comprising two branches of monomethoxy polyethylene glycol (mPEG), where the molecular mass of one of the branches of mPEG is between 10 kDa and 14 kDa, and the molecular mass of the other branch of mPEG is between 17 kDa and 23 kDa.

8. The method according to claim 7 wherein the molecular mass of one of the branches of mPEG is 12 kDa and the molecular mass of the other mPEG branch is 20 kDa.

9. The method according to claim 7 wherein the asymmetric branched polymeric structure is represented as:

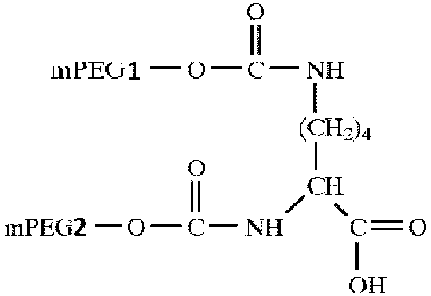

10. The method according to claim 9 wherein the mPEG1molecular mass is 12 kDa and mPEG2 molecular mass is 20 kDa, or the mPEG1molecular mass is 20 kDa and the mPEG2 molecular mass is 12 kDa.

11. The method according to any one of claims 7-10 wherein EPO is recombinant human EPO (rh EPO).
